(19) Europäisches Patentamt
European Patent Office
Office européen des brevets

(11) Veröffentlichungsnummer: 0 370 378 A2

(12) EUROPÄISCHE PATENTANMELDUNG

(21) Anmeldenummer: 89121170.8

(51) Int. Cl.5: C07D 209/52, A61K 31/40

(22) Anmeldetag: 16.11.89

Patentansprüche für folgende Vertragsstaaten: ES + GR.

(30) Priorität: 19.11.88 DE 3839127

(43) Veröffentlichungstag der Anmeldung: 30.05.90 Patentblatt 90/22

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Nickel, Wolf-Ulrich, Dr.
Robert-Stolz-Strasse 120
D-6232 Bad Soden am Taunus(DE)
Erfinder: Rüger, Wolfgang, Dr.
Parkstrasse 10
D-6233 Kelkheim (Taunus)(DE)
Erfinder: Urbach, Hansjörg, Dr.
Le Lavandoustrasse 41
D-6242 Kronberg/Taunus(DE)
Erfinder: Hock, Franz, Dr.
Altstadt 19
D-6110 Dieburg(DE)

(54) **Pyrrolidin-2-carbonsäure-Derivate mit psychotroper Wirkung.**

(57) Die vorliegende Erfindung betrifft Verbindungen der Formel I

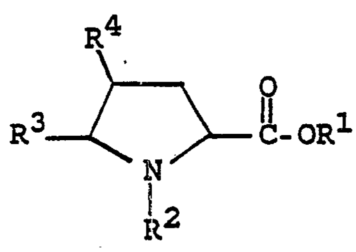

(I)

in welcher $R^1$ bis $R^4$ in der Beschreibung definiert sind, Verfahren zu deren Herstellung, deren Verwendung zur Behandlung und Prophylaxe cognitiver Dysfunktionen, sowie diese enthaltende Arzneimittel und Verfahren zu deren Herstellung.

EP 0 370 378 A2

## Pyrrolidin-2-carbonsäure-Derivate mit psychotroper Wirkung

Die Erfindung betrifft neue Pyrrolidin-2-carbonsäure-Derivate, diese enthaltende Mittel und deren Verwendung als Arzneimittel mit psychotroper Wirkung zur Behandlung und Propylaxe von Störungen im Zentralen Nervensystem, insbesondere mit nootroper Wirkung zur Behandlung cognitiver Dysfunktionen.

Der Erfindung liegt die Aufgabe zugrunde, neue Verbindungen mit antiamnestischer Wirkung zu finden.

Diese Aufgabe wird erfindungsgemäß gelöst durch die Verbindungen der Formel I

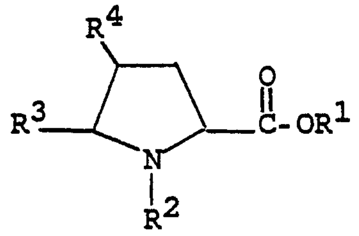

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,
einen gegebenenfalls substituierten ($C_3$-$C_8$)-Cycloalkyl-($C_1$-$C_8$)-alkanoyl-Rest,
einen gegebenenfalls substituierten ($C_7$-$C_{13}$)-Aroyl-Rest,
einen gegebenenfalls substituierten ($C_6$-$C_{12}$)-Aryl-($C_1$-$C_{18}$)-alkanoyl Rest,
einen gegebenenfalls substituierten ($C_7$-$C_{13}$)-Aroyl-($C_1$-$C_{18}$)-alkanoyl-Rest oder
einen gegebenenfalls substituierten ($C_1$-$C_{10}$)-Alkoxycarbonyl-Rest
bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten aromatischen Rest mit 6 - 12 C-Atomen oder
einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet, jedoch nicht Wasserstoff ist,
oder $R^4$ mit dem diesen Substituenten tragenden C-Atom ein spiroverknüpftes mono- oder bicyclisches Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, wobei Verbindungen, in denen gleichzeitig $R^1$

Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, sowie Verbindungen, in denen $R^2$ gegebenenfalls substituiertes $\alpha$-Amino-($C_1$-$C_6$)-alkanoyl bedeutet, ausgenommen sind.

Unter einem gegebenenfalls substituierten aliphatischen Rest versteht man einen aliphatischen acyclischen Rest, d.h. einen Rest mit einen offenen, geraden oder verzweigten Kohlenstoffkette, wie beispielsweise Alkyl, Alkenyl, Alkinyl und entsprechende mehrfach ungesättigte Reste. Er ist vorzugsweise unsubstituiert oder, beispielsweise durch Hydroxy, Alkoxy, Halogen, Amino, Alkanoylamino, Alkoxycarbonylamino, Arylalkoxycarbonylamino, Arylalkylamino,Alkylamino, Dialkylamino, Alkylthio, Arylthio, Carboxy, Carbamoyl, Alkoxycarbonyl, Alkanoyloxy, Alkoxycarbonyloxy, Aroyloxy oder Aryloxycarbonyloxy monosubstituiert.

Ein gegebenenfalls substituierter alicyclischer Rest und der über eine offene Kohlenstoffkette verknüpfte entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornanyl, Bornyl, Caranyl, Epibornyl, Epiisobornyl, Isobornyl, Menthanyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substiutierter aromatischer Rest ist vorzugsweise Aryl wie Phenyl, Biphenylyl oder Naphthyl, das gegebenenfalls wie auf Seite 6 bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein. Ein heteroaromatischer Rest und der entsprechende heteroaromatisch-aliphatische Rest können wie unten definiert, substituiert sein.

Unter einem gegebenenfalls substituierten araliphatischen Rest werden insbesondere Aralkylreste wie Arylalkyl, Diarylalkyl, Indanyl oder Fluorenyl verstanden, worin Aryl wie unten definiert ist und in der dort angegebenen Weise substituiert sein kann.

$R^3$ und $R^4$ kann mit den diese tragenden Atomen ein mono-, oder bicyclisches alicyclisches oder aromatisches Ringsystem mit 3 - 8 C-Atomen bilden. Als Ringsytem kommen in Betracht Cyclopropan, Cyclobutan, Cyclopentan, Cyclohexan, Cycloheptan, Cyclooctan, Norbornan, Bicyclo[2.2.2]octan, Bicyclo[3.2.1]octan, Benzol, die teilweise ungesättigt und/oder substituiert sein können.

Im Falle, daß $R^4$ mit dem diesen Substituenten tragenden Atom ein spiroverknüpftes mono- oder bicyclisches Ringsystem bildet, kommen für monocyclische Substituenten alicyclische oder heterocyclische Ringsysteme mit 3 - 8 Atomen, in denen bis zu 2 Sauerstoffatome oder bis zu 2 Schwefelatome oder ein Stickstoffatom vorkommen kann, und für bicyclische Substituenten vorzugsweise alicyclische Ringssysteme mit 5 - 9 Atomen in Betracht.

Bei Verbindungen der Formel 1, die mehrere chirale Atome besitzen, kommen alle möglichen Diastereomere als Racemat oder Enantiomere, oder Gemische verschiedener Diastereomere in Betracht. Bevorzugt sind jedoch die Verbindungen der Formel I, in denen die chiralen C-Atome S-Konfiguration aufweisen.

Eine bevorzugte Ausführungsform ist gekennzeichnet durch Verbindungen der Formel I, in welcher

$R^1$ Wasserstoff;

($C_1$-$C_{18}$)-Alkyl;

einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;

einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;

($C_6$-$C_{12}$)-Aryl, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder

Sulfamoyl mono-, di- oder trisubstituiert sein kann;
Amino-($C_1$-$C_8$)-alkyl;
($C_1$-$C_4$)-Alkanoylamino-($C_1$-$C_8$)-alkyl;
($C_7$-$C_{13}$)-Aroylamino-($C_1$-$C_8$)-alkyl;
($C_1$-$C_4$)-Alkoxycarbonylamino-($C_1$-$C_8$)-alkyl;
($C_6$-$C_{12}$)-Aryl-($C_1$-$C_4$)-alkoxycarbonylamino-($C_1$-$C_8$)-alkyl
($C_6$-$C_{12}$)-Aryl-($C_1$-$C_4$)-alkylamino-($C_1$-$C_8$)-alkyl;
($C_1$-$C_4$)-Alkylamino-($C_1$-$C_8$)-alkyl;
Di-($C_1$-$C_4$)-alkylamino-($C_1$-$C_8$)-alkyl;
Guanidino-($C_1$-$C_8$)-alkyl;
($C_1$-$C_4$)-Alkylthio-($C_1$-$C_8$)-alkyl;
($C_6$-$C_{12}$)-Arylthio-($C_1$-$C_8$)-alkyl oder ($C_6$-$C_{12}$)-Aryloxy- ($C_1$-$C_8$)-alkyl, die beide im Arylteil wie oben beschrieben substituiert sein können;
Carboxy-($C_1$-$C_{17}$)-alkyl, ($C_1$-$C_4$)-Alkoxycarbonyl-($C_1$-$C_{17}$)-alkyl, Carbamoyl-($C_1$-$C_{17}$)-alkyl, ($C_1$-$C_4$)-Mono- oder dialkylcarbamoyl-($C_1$-$C_{17}$)-alkyl, wobei Alkyl gegebenenfalls durch ($C_6$-$C_{12}$)-Aryl substituiert ist, bedeutet;
$R^2$ Wasserstoff;
($C_1$-$C_{18}$)-Alkyl;
einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenwasserstoffrest der Formel $C_cH_{2c-d-1}$, worin c für eine ganze Zahl 3 bis 20 und d für eine gerade Zahl 0 bis (c-2) stehen;
($C_6$-$C_{12}$)-Aryl;
($C_6$-$C_{12}$)-Aryl-($C_1$-$C_8$)-alkyl oder ($C_7$-$C_{13}$)-Aroyl-($C_1$-$C_8$)-alkyl, die im Arylteil wie oben beschrieben substituiert sein können;
mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und I bis 2 Ringatome Schwefel oder Sauerstoff und/oder 1 bis 4 Ringatome Stickstoff darstellen, die im Heteroarylteil wie oben bei Aryl beschrieben substituiert sein können;
($C_1$-$C_{18}$)-Alkanoyl;
($C_6$-$C_{12}$)-Aryl-($C_1$-$C_8$)-alkanoyl, das im Arylteil wie oben beschrieben substituiert sein kann,
($C_7$-$C_{13}$)-Aroyl-($C_1$-$C_8$)-alkanoyl, das im Arylteil wie oben beschrieben substituiert sein kann,
($C_1$-$C_6$)-Alkoxycarbonyl-($C_1$-$C_8$)alkanoyl,
($C_6$-$C_{12}$)-Aryloxycarbonyl-($C_1$-$C_8$)alkanoyl, das im Arylteil wie oben beschrieben substituiert sein kann;
($C_6$-$C_{12}$)-Aryl-($C_1$-$C_8$)-alkoxycarbonyl;
($C_1$-$C_6$)-Alkoxy-($C_1$-$C_6$)-alkanoyl
($C_6$-$C_{12}$)-Aryloxy-($C_1$-$C_6$)-alkanoyl, das im Arylteil wie oben bei Aryl beschrieben substituiert sein kann,
($C_1$-$C_6$)-Acyl-($C_1$-$C_8$)-alkanoyl,
Carboxy-($C_1$-$C_4$)-alkanoyl,
Carbamoyl-($C_1$-$C_4$)-alkanoyl,
Amino-($C_1$-$C_4$)-alkyl;
($C_1$-$C_4$)-Alkanoylamino-($C_1$-$C_4$)-alkyl;
($C_7$-$C_{13}$)-Aroylamino-($C_1$-$C_4$)-alkyl;
($C_1$-$C_4$)-Alkoxycarbonylamino-($C_1$-$C_4$)-alkyl;
($C_6$-$C_{12}$)-Aryl-($C_1$-$C_4$)-alkoxycarbonylamino-($C_1$-$C_4$)-alkyl;
($C_6$-$C_{12}$)-Aryl-($C_1$-$C_4$)-alkylamino-($C_1$-$C_4$)-alkyl;
($C_1$-$C_4$)-Alkylamino-($C_1$-$C_4$)-alkyl;
Di-($C_1$-$C_4$)-alkylamino-($C_1$-$C_4$)-alkyl;
Guanidino-($C_1$-$C_4$)-alkyl;
($C_1$-$C_4$)-Alkylthio-($C_1$-$C_4$)-alkyl;
($C_6$-$C_{12}$)-Arylthio-($C_1$-$C_4$)-alkyl, das im Arylteil wie oben beschrieben substituiert sein kann;
Carboxy-($C_1$-$C_4$)-alkyl; Carbamoyl-($C_1$-$C_4$)-alkyl;
($C_1$-$C_4$)-Alkoxycarbonyl-($C_1$-$C_4$)-alkyl;
($C_6$-$C_{12}$)-Aryloxy-($C_1$-$C_4$)-alkyl, das im Arylteil wie oben bei Aryl beschrieben substituiert sein kann, bedeutet
und
$R^3$ und $R^4$ zusammen mit den sie tragenden Atomen ein gegebenenfalls substituiertes mono- oder bicyclisches Ringsystem von 3 - 8 C-Atomen bilden,wobei Verbindungen, in denen gleichzeitig $R^1$

Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, ausgenommen sind.

Insbesondere seien Verbindungen der Formel I genannt, in welcher

$R^1$ Wasserstoff; $(C_1-C_8)$-Alkyl; $(C_2-C_6)$-Alkenyl;
$(C_3-C_9)$-Cycloalkyl; Amino-$(C_1-C_4)$-alkyl;
$(C_2-C_5)$-Acylamino-$(C_1-C_4)$-alkyl;
$(C_7-C_{13})$-Aroylamino-$(C_1-C_4)$-alkyl;
$(C_1-C_4)$-Alkoxycarbonylamino-$(C_1-C_4)$-alkyl;
$(C_1-C_4)$-Alkoxycarbonyl-$(C_1-C_{10})$-alkyl;
Carbamoyl-$(C_1-C_{10}$-alkyl; $(C_1-C_4)$-Mono oder -Dialkylcarbamoyl-$(C_1-C_{10})$-alkyl, wobei Alkyl gegebenenfalls durch Phenyl substituiert ist;
$(C_6-C_{12})$-Aryl-$(C_1-C_6)$-alkyl;
$(C_6-C_{12})$-Aryl-$(C_1-C_4)$-alkoxycarbonylamino-$(C_1-C_4)$-alkyl;
$(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di-oder trisubstituiert sein kann;
insbesondere Wasserstoff, Methyl, Ethyl, Octyl, Benzyl, Benzhydryl, tert. Butoxycarbonylamino-$(C_1-C_4)$-alkyl, Benzyloxycarbonylamino-$(C_1-C_4)$-alkyl,
Carboxy-$(C_1-C_{10})$-alkyl,
$(C_1-C_4)$-Alkoxycarbonyl-$(C_1-C_{10})$-alkyl bedeutet;

$R^2$ Wasserstoff;
$(C_1-C_6)$-Alkyl, das gegebenenfalls durch Amino, $(C_1-C_6)$-Acylamino oder Benzoylamino substituiert sein kann;
$(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann;
$(C_6-C_{12})$-Aryl-$(C_1-C_6)$-alkyl oder $(C_7-C_{13})$-Aroyl-$(C_1-C_4)$-alkyl, die beide wie vorstehend definiert im Arylrest substituiert sein können;
$(C_1-C_8)$-Alkanoyl;
$(C_6-C_{12})$-Aryl-$(C_1-C_6)$-alkanoyl, das wie vorstehend definiert im Arylteil substituiert sein kann;
$(C_1-C_4)$-Alkoxycarbonyl-$(C_1-C_7)$-alkanoyl;
$(C_6-C_{12})$-Aryloxycarbonyl-$(C_1-C_7)$-alkanoyl, das wie vorstehend definiert im Arylteil substituiert sein kann;
$(C_1-C_4)$-Acyl-$(C_2-C_6)$-alkanoyl;
$(C_1-C_4)$-Acylamino-$(C_1-C_6)$-alkanoyl;
$(C_7-C_{13})$-Aroyl-$(C_1-C_6)$-alkanoyl, das wie vorstehend definiert im Arylteil substituiert sein kann;
insbesondere aber
Wasserstoff, $(C_1-C_6)$-Alkanoyl, $(C_6-C_{12})$-Aryl-$(C_1-C_6)$-alkanoyl, $(C_7-C_{13})$-Aroyl-$(C_1-C_3)$-alkanoyl, tert. Butoxycarbonyl, Benzyloxycarbonyl, tert. Butoxycarbonylamino-$(C_2-C_3)$-alkanoyl, Benzyloxycarbonylamino-$(C_2-C_3)$-alkanoyl, $(C_6-C_{12})$-Aryl-$(C_1-C_3)$-acylamino-$(C_2-C_3)$-alkanoyl, Trifluoracetylamino-$(C_2-C_3)$-alkanoyl bedeutet;

$R^3$ und $R^4$ zusammen mit den sie tragenden Atomen, ein monocyclisches alicyclische oder aromatisches Ringsystem, insbesondere Cyclopentan, Cyclopenten, Cyclohexan, Cycloctan und Benzol, bilden, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopenten- oder Cyclopentenring bedeuten, sowie Verbindungen in denen $R^2$ $\alpha$-Acylamino-$(C_1-C_6)$alkanoyl bedeutet, ausgenommen sind.

Die Erfindung betrifft weiterhin ein Verfahren zur Herstellung einer Verbindung der Formel I, das dadurch gekennzeichnet ist, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel I mit freier(en) Carboxylgruppe(n) gegebenenfalls verestert und die erhaltenen Verbindungen gegebenenfalls in ihre physiologisch verträglichen Salze überführt.

In der genannten Weise kann man beispielsweise Verbindungen der Formeln II und III

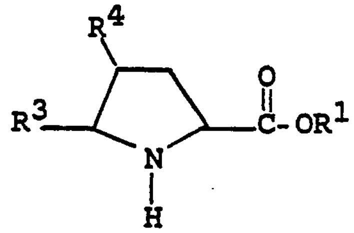

$R^2 - X$

II III

unter Bildung von Verbindungen der Formel I umsetzen, worin $R^1$ bis $R^4$ in der oben angegebenen Weise definiert sind, und X im Falle von Alkylierungen eine geeignete Abgangsgruppe, insbesondere Halogen, ($C_1$-$C_4$)-Alkylsulfonyloxy, Arylsulfonyloxy, darstellt, im Falle von Acylierungen Hydroxy, ($C_1$-$C_3$)-Alkoxy, ($C_6$-$C_{12}$)-Aryloxy, ($C_6$-$C_{12}$)-Aryl-($C_1$-$C_6$)alkoxy oder den Rest eines aktivierten Säurederivats bedeutet.

Die Umsetzung dieser Verbindungen kann beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonate in einem Lösungsmittel wie $CH_3CN$ durchgeführt werden. Aminogruppen in Verbindungen der Formel II können mit Tetraethyldiphosphit aktiviert werden. Die Verbindungen der Formel III können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Houben-Weyl, Meth. d.organ. Chemie Bd. XV 1 und 2). Die Reaktion wird vorzugsweise zwischen -20 °C und dem Siedepunkt des Reaktionsgemisches durchgeführt.

Die Umsetzung zu Verbindungen der Formel I kann aber auch thermisch in einem geeigneten organischen Lösungsmittel im Temperaturbereich zwischen 0 °C und dem Siedepunkt des Lösungsmittels erfolgen.

Die Herstellung der neuen Verbindungen der allgemeinen Formel I kann auch unter Anwendung dem Fachmann vertrauter Alkylierungsmethoden bzw. Veresterungsmethoden erfolgen, beispielsweise die Umsetzung einer Verbindung der Formel I, worin der Rest $R^1$ Wasserstoff bedeutet, mit einem entsprechenden Alkylierungsmittel der allgemeinen Formel $R^1X$, worin X wie oben beschrieben definiert ist, wie z. B. einem Alkylhalogenid, unter basischer Katalyse in einem polar protischen oder dipolar aprotischen Lösungsmittel, oder beispielsweise die Umsetzung einer Verbindung der Formel I mit z. B. einem aliphatischen Alkohol unter saurer oder basischer Katalyse in einer polar protischen oder dipolar aprotischen Lösungsmittel.

Die nootrope Wirkung der erfindungsgemäßen Verbindungen wurde an Mäusen, die ein Körpergewicht von 20 - 25 g besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, 0. BURESOVA und J. HUSTON in "Techniques and Basic Experiments for the Study of Brain and Behavior", Elsevier Scientific Publishers, Amsterdam (1983) beschrieben.
Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam).

Die deutliche Überlegenheit der erfindungsgemäßen Verbindungen über die Vergleichssubstanz zeigte sich darin, daß die Scopolamin-induzierte Amnesie im inhibitory avoidance-Test sich mit einer MED (minimal effective Dosis) von 0,1 - 30 mg/kg p.o. aufheben läßt. Die Vergleichssubstanz hat eine MED von ca. 500 - 1000 mg/kg p.o.

Die erfindungsgemäßen Verbindungen haben im allgemeinen nur eine geringe Toxizität und sind aufgrund ihrer pharmakologischen Eigenschaften für die Behandlung cognitiver Dysfunktionen unterschiedlicher Genese, wie sie z.B. bei der Alzheimer'schen Krankheit oder der senilen Demenz auftreten, geeignet.

Es wurde überraschenderweise gefunden, daß die bereits als Zwischenprodukte bekannten Verbindungen der Formel I, in denen $R^2$ die erforderlichenfalls geschützte Seitenkette einer natürlich vorkommenden α-Aminosäure bzw. gegebenenfalls substituiertes α-Amino-($C_1$-$C_6$)-alkanoyl bedeutet, sowie die ebenfalls bereits als Zwischenprodukt bekannten Verbindungen der Formel I, in deren gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, die gleichen guten pharmakologischen Eigenschaften bei

geringer Toxizität aufweisen. Die vorliegende Erfindung betrifft daher auch diese bereits als Zwischenprodukte bekannten Verbindungen der Formel I als pharmakologische Wirkstoffe.

Falls $R^2$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen in der Peptidchemie übliche Gruppen bevorzugt (vgl. Houben-Weyl, Bd. XV/1 und XV/2). Im Falle, daß $R^2$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alk, insbesondere Methyl oder Ethyl in Frage.

Bevorzugte Seitenketten von natürlich vorkommenden α-Aminosäuren sind solche von proteinogenen α-Aminosäuren.

Die Erfindung umfaßt weiterhin Verbindungen der Formel I enthaltende Arzneimittel, Verfahren zu deren Herstellung sowie die Verwendung der Verbindungen der Formel I bei der Herstellung von Arzneimitteln, die zur Behandlung und Prophylaxe der vorstehend genannten Krankheiten eingesetzt werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die folgenden Beispiele sollen die Erfindung erläutern, ohne sie auf die genannten Verbindungen zu beschränken.

**Beispiel 1**

2-N-(4-(2-Naphthyl)butyryl)-(1S,3S,5S)-2-azabicyclo[3.3.0]octan-3-carbonsäurebenzylester

In 40 ml DMF wurden 2,5 g (1S,3S,5S)-2-azabicyclo[3.3.0] octan-3-carbonsäurebenzylester und 2,2 g 4-(2-Naphthyl)-buttersäure gelöst und auf -5 °C₁ gekühlt. Dazu wurden unter Stickstoffatmosphäre 7,7 ml Triethylamin und nachfolgend 10 ml n-Propylphosphonsäureanhydrid (50 %ige Lösung in Dichlormethan) zupipettiert. Diese Mischung wurde bis zur vollständigen Umsetzung 48 Stunden bei Raumtemperatur gerührt. Die Lösung wurde mit 100 ml Essigester verdünnt und der Reihe nach je zweimal mit gesättigter $NaHCO_3$-Lösung, 10 %ige Zitronensäurelösung und gesättigter NaCl-Lösung extrahiert. Nach Trocknen mit $MgSO_4$ und Einengen der organischen Phase wurde das erhaltene Rohprodukt säulenchromatographisch. über Kieselgel (Cyclohexan/Essigester) aufgetrennt. Man erhielt 2,52 g des gewünschten Produktes.
$\alpha_D^{25}$ : -13,9 ° (c=1,01 in Methanol).

Folgende Verbindungen wurden analog der in Beispiel 1 beschriebenen Vorschrift hergestellt:

**Beispiel 2**

2-N-(5-Phenylvaleryl)-(1S,3S,5S)-2-azabicyclo[3.3.0]octan-3-carbonsäurebenzylester

$\alpha_D^{25}$ : -16,2 ° (c = 1,18 in Methanol).

**Beispiel 3**

2-N-(3-(2-Naphthoyl)-propionyl)-(1S,3S,5S)-2-azabicyclo[3.3.0]octan-3-carbonsäurebenzylester

$\alpha_D^{25}$ : -20,3 ° (c = 1,08 in Methanol).

**Beispiel 4**

2-Phenylacetyl-(1S,3S,5S)-2-azabicyclo[3.3.0]octan-3-carbonsäurebenzylester

$\alpha_D^{25}$ : -26,4 ° (c = 1 in Methanol).

**Beispiel 5**

2-(3-Phenylpropionyl)-(1S,3S,5S)-2-azabicyclo[3.3.0]octan-3-carbonsäurebenzylester

$\alpha_D^{25}$ : -16,5 ° (c = 1 in Methanol).

**Beispiel 6**

2-(4-Phenylbutyryl)-(1S,3S,5S)-2-azabicyclo[3.3.0]octan-3-carbonsäurebenzylester

$\alpha_D^{25}$ : -15,3 ° (c = 1 in Methanol).

**Beispiel 7**

2-(5-Phenylhexanoyl)-(1S,3S,5S)-2-azabicyclo[3.3.0]octan-3-carbonsäurebenzylester

$\alpha_D^{25}$ : -12,6 ° (c = 1 in Methanol).

**Beispiel 8**

2-N-(N-Trifluoracetyl-S-alanyl)-(1S,3S,5S)-2-azabicyclo[3.3.0]octan-3-carbonsäurebenzylester

$\alpha_D^{20}$ : -64,9 ° (c = 1,0 in Methanol).
Schmelzpunkt: 75 - 78 °C.

**Beispiel 9**

2-Propionyl-(1S,3S,5S)-2-azabicyclo[3.3.0]octan-3-carbonsäure-n-octylester

$\alpha_D^{25}$ : -8,4 ° (c = 1 in Methanol).

**Beispiel 10**

2-Tert.butyloxycarbonyl-(1S,3S,5S)-2-azabicyclo[3.3.0]octan-3-carbonsäure-n-octylester

$\alpha_D^{25}$ : +5,5 ° (c=2 in Methanol).

**Beispiel 11**

2-(6-Phenylhexanoyl)-(1S,3S,5S)-2-azabicyclo[3.3.0]octan-3-carbonsäure-n-octylester

$\alpha_D^{25}$ : - 3,7 ° (c=2 in Methanol).

**Beispiel 12**

2-[N-Tert.butyloxycarbonyl-S-alanyl]-(1S,3S,5S)-2-azabicyclo[3.3.0]octan-3-carbonsäure-n-octylester

$\alpha_D^{25}$ : -39,9 ° (c=1 in Methanol).

**Beispiel 13**

2-[N-Benzyloxycarbonyl-S-alanyl)-(1S,3S,5S)-2-azabicyclo[3.3.0]octan-3-carbonsäure-n-octylester

$\alpha_D^{25}$ : -38,0 ° (c=1 in Methanol).

**Beispiel 14**

2-[N-(3-Phenylpropionyl)-S-alanyl]-(1S,3S,5S)-2-azabicyclo[3.3.0]octan-3-carbonsäure-n-octylester

$\alpha_D^{25}$ : -35,3 ° (c=1 in Methanol)

**Beispiel 15**

2-N-(4-(2-Naphthyl)-butyryl)-(1S,3S,5S)-2-azabicyclo[3.3.0]octan-3-carbonsäure

2 g des in Beispiel 1 beschriebenen Benzylesters wurden in 60 ml absolutem Ethanol gelöst und bei Raumtemperatur katalytisch über Palladium-Kohle hydriert. Nach beendeter Reaktion wurde vom Katalysator abfiltriert und das Filtrat im Vakuum vom Lösungsmittel befreit. Es wurden 1,52 g Produkt als amorphes Pulver erhalten.
$\alpha_D^{25}$ : +14,2 ° (c=1,06 in Methanol).
Die folgenden Verbindungen der Beispiele 16 - 19 wurden analog der in Beispiel 15 angegebenen Vorschrift hergestellt

**Beispiel 16**

2-N-(5-Phenylvaleryl)-(1S,3S,5S)-2-azabicyclo[3.3.0]octan-3-carbonsäure-dicyclohexylammoniumsalz

Der im Beispiel 2 beschriebene Benzylester wurde wie in Beispiel 15 behandelt, die erhaltene Carbonsäure in Diisopropylether gelöst und als Dicyclohexylammonium-Salz gefällt.
$\alpha_D^{20}$ : +18,7 ° (c=1,04 in Methanol).
Schmelzpunkt: 113 °C

**Beispiel 17**

2-N-(3-(2-Naphthoyl)-propionyl)-(1S,3S,5S)-2-azabicyclo[3.3.0]octan-3-carbonsäure

Schmelzpunkt: 147 °C

**Beispiel 18**

2-(N-(3-Phenylpropionyl)-S-alanyl]-(1S,3S,5S)-2-azabicyclo[3.3.0]octan-3-carbonsäure

$\alpha_D^{20}$ : -25,2 ° (c = 1,02 in Methanol).
Schmelzpunkt: 180 - 184 °C

**Beispiel 19**

2-N-(Tert.butoxycarbonyl)-S-alanyl)-(1S,3S,5S)-2-azabicyclo[3.3.0]octan-3-carbonsäure

$\alpha_D^{25}$ : -29,3 ° (c = 1 in Methanol).

**Beispiel 20**

2-N-(4-(2-Naphthyl)-butyryl)-(1S,3S,5S)-2-azabicyclo-[3.3.0]octan-3-carbonsäure-n-octylester

0,5 g der in Beispiel 8 erhaltenen Carbonsäure wurden in 10 ml absolutem Dimethylformamid gelöst, anschließend für eine Stunde unter Feuchtigkeitsausschluß mit 285 mg $KHCO_3$ bei 40 °C behandelt und danach mit 0,6 ml n-Octylbromid versetzt. Nach 3 Stunden bei 40 °C war die Reaktion beendet. Die Lösung wurde mit Wasser verdünnt und dreimal mit je 20 ml Essigester extrahiert. Die organische Phase wurde mit NaCl-Lösung gewaschen, mit $MgSO_4$ getrocknet und im Vakuum eingeengt. Das so gewonnene Rohprodukt wurde durch Säulenchromotographie auf Kieselgel (Cyclohexan/Essigester) gereinigt. Die Ausbeute betrug 378 mg.
$\alpha_D^{20}$ : -2,4 ° (c = 1,14 in Methanol).

Die folgenden Verbindungen der Beispiele 21 und 22 wurden analog zu der obigen in Beispiel 20 beschriebenen Vorschrift erhalten:

**Beispiel 21**

2-Phenylacetyl-(1S,3S,5S)-2-azabicyclo[3.3.0]octan-3-carbonsäure-n-octylester

$\alpha_D^{25}$ : -9,8 ° (c = 1 in Methanol).

**Beispiel 22**

2-(5-Phenylvaleryl)-(1S,3S,5S)-2-azabicyclo[3.3.0]octan-3-carbonsäure-n-octylester

$\alpha_D^{25}$ : -4,0 ° (c = 0,99 in Methanol).

**Beispiel 23**

2-(N-(3-Phenylpropionyl)-S-alanyl)-(1S,3S,5S)-2-azabicyclo[3.3.0]octan-3-carbonsäurebenzylester

a) N-(3-Phenylpropionyl)-S-alaninbenzylester 9,70 g L-Alaninbenzylesterhydrochlorid und 6,75 g 3-Phenylpropionsäure wurden in 125 ml Dichlormethan gelöst, auf -5 °C abgekühlt und mit 37,5 ml Triethylamin unter $N_2$ versetzt. Nach 10 Minuten wurden zu dieser Lösung 45 ml n-Propylphosphonsäure-

anhydrid (50 %ig in $CH_2Cl_2$) zugetropft, eine Stunde bei -5 °C und anschließend bei Raumtemperatur über Nacht gerührt. Die

Aufarbeitung erfolgte wie in Beispiel 1 und ergab nach Umkristallisation aus Cyclohexan/Essigester 9,5 g gewünschtes Produkt vom Schmelzpunkt 59 - 60 °C.

b) Der unter a) erhaltene Benzylester wurde in 250 ml Ethanol über Palladium-Kohle katalytisch hydriert.

Nach Abfiltrieren vom Katalysator und Einengen im Vakuum wurden 6,9 g N-(3-Phenylpropionyl)-S-alanin erhalten.

$\alpha_D^{20}$ : -32,9 ° (c = 1,66 in Methanol).

c) 3,3 g der unter b) erhaltenen Carbonsäure und 3,65 g 2-Azabicyclo[3.3.0]octan-3-carbonsäurebenzylester wurden nach dem in Beispiel 1 beschriebenen Verfahren mit 15 ml n-Propylphosphonsäureanhydrid umgesetzt, wie beschrieben aufgearbeitet und isoliert. So erhielt man 3,3 g des gewünschten Produktes.

$\alpha_D^{20}$ : -43,3 ° (c = 1,21 in Methanol).

**Beispiel 24**

(1S,3S,5S)-2-Azabicyclo[3.3.0]octan-3-carbonsäure-n-octylester-L-hydrogentartrat

4,5 g (12,2 mmol) Boc-Verbindung aus Beispiel 10 wurden bei 0 °C mit 15 ml Trifluoressigsäure übergossen und 90 Minuten gerührt. Das Lösungsmittel wurde abgedampft, der Rückstand in gesättigter Natriumbicarbonatlösung aufgenommen, dreimal mit Essigester extrahiert, die vereinigten Extrakte getrocknet und eingeengt. Man erhielt 3,1 g (1S,3S,5S)-2-Azabicyclo[3.3.0]octan-3-carbonsäure-n-octylester.

Dieser wird in Ether gelöst und eine Lösung von 1,74 g L-Weinsäure in Aceton zugesetzt. Man saugt den Niederschlag ab und erhält 2,5 g des Produktes.

$\alpha_D^{25}$ : -13,6 ° (c = 1 in Methanol).

**Beispiel 25**

(1S,3S,5S)-2-Azabicylco[3.3.0]octan-3-carbonsäure-n-octylester-hydrochlorid

Zu einer Suspension von 10 g (64 mmol) (1S,3S,5S)-2-Azabicyclo[3.3.0]octan-3-carbonsäure in 100 ml destilliertem n-Octanol tropft man bei 40 °C 16,3 g (150 mmol) destilliertes Trimethylsilylchlorid und rührt über Nacht bei 40 °C. Die flüchtigen Bestandteile werden am Rotationsverdampfer entfernt, das Octanol durch Kurzwegdestillation im Hochvakuum abgetrennt, der Destillationsrückstand in Methylenchlorid aufgenommen, eingeengt und zweimal mit Diisopropylether verrieben. Man erhält 14,6 g (5 %) der Titelverbindung.

Schmp. 76-78 °C

$\alpha_D^{25}$ = -23,7° (c = 1, Methanol).

**Beispiel 26**

2-(2Z-3-Methoxycarbonyl-propenoyl)-(1S,3S,5S)-2-azabicyclo[3.3.0]octan-3-carbonsäure-n-octylester

Diese Verbindung wurde analog dem in Beispiel 1 beschriebenen Verfahren aus (1S,3S,5S)-2-azabicyclo[3.3.0]octan-3-carbonsäure-n-octylester und Maleinsäure-monomethylester erhalten.

MS (DCI) = 380 (M + 1).

**Beispiel 27**

2-(2Z-3-n-Octyloxycarbonyl-propanoyl)-(1S,3S,5S)-2-azabicyclo[3.3.0]octan-3-carbonsäure-n-octylester

Die Verbindung wurde analog dem in Beispiel 1 beschriebenen Verfahren aus (1S,3S,5S)-2-azabicyclo-[3.3.0]octan-3-carbnonsäure-n-octylester und Maleinsäure-mono-n-octylester erhalten.
$[\alpha]_D^{25}$ = -22,1 °. (c = 1, Methanol).

**Beispiel 28**

2-Maleoyl-(1S,3S,5S)-2-azabicyclo[3.3.0]octan-3-carbonsäure-n-octylester

1,03 g (2,7 mmol) des in Beispiel 26 beschriebenen Diesters wurden in 10 ml Dioxan gelöst, eine Lösung von 0,126 g (5,4 mmol) Lithiumhydroxid in 10 ml Wasser zugesetzt und drei Stunden bei Raumtemperatur gerührt. Die Lösung wurde auf pH 3 angesäuert, mit Essigester extrahiert, der Extrakt getrocknet, eingeengt und das Rohprodukt durch Säulenchromatographie an Kieselgel (Laufmittel Methanol/Methylenchlorid 3:97) gereinigt. Ausbeute: 0,5 g der Titelverbindung.
$[\alpha]_D^{25}$ = -19,4° (c = 1, Methanol).

**Beispiel 29**

(1S,3S,5S)-2-Azabicyclo[3.3.0]octan-3-carbonsäure-n-octylester-hydrogenmaleinat

Diese Verbindung wurde aus (1S,3S,5S)-2-Azabicyclo-[3.3.0]octan-3-carbonsäure-n-octylester und Maleinsäure in Ether/Aceton 5/1 erhalten. $[\alpha]_D^{25}$ : -16,1° (c = 1 in Methanol).

**Ansprüche**

1. Verbindung der Formel I

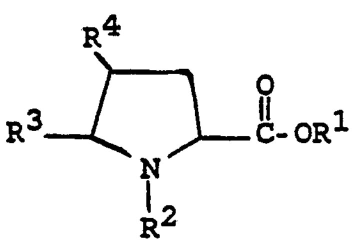

(I)

in welcher
$R^1$ Wasserstoff,
einen gegebenenfalls. substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,

einen gegebenenfalls substituierten ($C_3$-$C_8$)-Cycloalkyl-($C_1$-$C_8$)-alkanoyl-Rest, einen gegebenenfalls substituierten ($C_7$-$C_{13}$)-Aroyl-Rest, einen gegebenenfalls substituierten ($C_6$-$C_{12}$)-Aryl-($C_1$-$C_{18}$)-alkanoyl Rest, einen gegebenenfalls substituierten ($C_7$-$C_{13}$)-Aroyl-($C_1$-$C_{18}$)-alkanoyl-Rest oder einen gegebenenfalls substituierten ($C_1$-$C_{10}$)-Alkoxycarbonyl-Rest bedeutet,
$R^3$ einen gegebenenfalls substituierten aliphatischen Rest mit 1 - 18 C-Atomen, einen gegebenenfalls substituierten alicyclischen Rest mit 3 - 20 C-Atomen, einen gegebenenfalls substituierten aromatischen Rest mit 6 - 12 C-Atomen oder einen gegebenenfalls substituierten araliphatischen Rest mit 7 - 32 C-Atomen bedeutet,
$R^4$ einen gegebenenfalls substituierten aliphatischen Rest mit 1 - 18 C-Atomen, einen gegebenenfalls substituierten alicyclischen Rest mit 3 - 20 C-Atomen, einen gegebenenfalls substituierten alicyclisch-aliphatischen Rest mit 4 - 20 Atomen, einen gegebenenfalls substituierten aromatischen Rest mit 6 - 12 C-Atomen, einen gegebenenfalls substituierten araliphatischen Rest mit 7 - 32 C-Atomen, einen gegebenenfalls substituierten heteroaromatischen oder heteroaromatisch-($C_1$-$C_8$)-aliphatischen Rest mit 5 - 12 Ringatomen bedeutet, jedoch nicht Wasserstoff ist, oder $R^4$ mit dem diesen Substituenten tragenden C-Atom ein spiroverknüpftes mono- oder bicyclisches Ringsystem von 3 - 8 C-Atomen bildet, oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, sowie Verbindungen, in denen $R^2$ gegebenenfalls substituiertes α-Amino-($C_1$-$C_6$)-alkanoyl bedeutet, ausgenommen sind.

2. Verbindung der Formel I, gemäß Anspruch 1, dadurch gekennzeichnet, daß
$R^1$ Wasserstoff;
($C_1$-$C_{18}$)-Alkyl;
einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;
($C_6$-$C_{12}$)-Aryl, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)- Alkanoylamino, Methylendioxy, Carboxy, Cyano und/oder Sulfamoyl mono-, di- oder trisubstituiert sein kann;
Amino-($C_1$-$C_8$)-alkyl;
($C_1$-$C_4$)-Alkanoylamino-($C_1$-$C_8$)-alkyl;
($C_7$-$C_{13}$)-Aroylamino-($C_1$-$C_8$)-alkyl;
($C_1$-$C_4$)-Alkoxycarbonylamino-($C_1$-$C_8$)-alkyl;
($C_6$-$C_{12}$)-Aryl-($C_1$-$C_4$)-alkoxycarbonylamino-($C_1$-$C_8$)-alkyl;
($C_6$-$C_{12}$)-Aryl-($C_1$-$C_4$)-alkylamino-($C_1$-$C_8$)-alkyl;
($C_1$-$C_4$)-Alkylamino-($C_1$-$C_8$)-alkyl;
Di-($C_1$-$C_4$)-alkylamino-($C_1$-$C_8$)-alkyl;
Guanidino-($C_1$-$C_8$)-alkyl;
($C_1$-$C_4$)-Alkylthio-($C_1$-$C_8$)-alkyl;
($C_6$-$C_{12}$)-Arylthio-($C_1$-$C_8$)-alkyl oder ($C_6$-$C_{12}$)-Aryloxy-($C_1$-$C_8$)-alkyl, die beide im Arylteil wie oben beschrieben substituiert sein können;
Carboxy-($C_1$-$C_{17}$)-alkyl, ($C_1$-$C_4$)-Alkoxycarbonyl-($C_1$-$C_{17}$)-alkyl, Carbamoyl-($C_1$-$C_{17}$)-alkyl, ($C_1$-$C_4$)-Mono- oder di-alkylcarbamoyl-($C_1$-$C_{17}$)-alkyl, wobei Alkyl gegebenenfalls durch ($C_6$-$C_{12}$)-Aryl substituiert ist, bedeutet;
$R^2$ Wasserstoff;
($C_1$-$C_{18}$)-Alkyl;
einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenwasserstoffrest der Formel $C_cH_{(2c-d-1)}$, worin c für eine ganze Zahl 3 bis 20 und d für eine gerade Zahl 0 bis (c-2) stehen;

$(C_6-C_{12})$-Aryl;
$(C_6-C_{12})$-Aryl-$(C_1-C_8)$-alkyl oder $(C_7-C_{13})$-Aroyl-$(C_1-C_8)$-alkyl, die im Arylteil wie oben beschrieben substituiert sein können;
mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroarylteil wie oben bei Aryl beschrieben substituiert sein können;
$(C_1-C_{18})$-Alkanoyl;
$(C_6-C_{12})$-Aryl-$(C_1-C_8)$-alkanoyl, das im Arylteil wie oben beschrieben substituiert sein kann,
$(C_7-C_{13})$-Aroyl-$(C_1-C_8)$-alkanoyl, das im Arylteil wie oben beschrieben substituiert sein kann,
$(C_1-C_6)$-Alkoxycarbonyl-$(C_1-C_8)$alkanoyl,
$(C_6-C_{12})$-Aryloxycarbonyl-$(C_1-C_8)$alkanoyl, das im Arylteil wie oben beschrieben substituiert sein kann;
$(C_6-C_{12})$-Aryl-$(C_1-C_8)$-alkoxycarbonyl;
$(C_1-C_6)$-Alkoxy-$(C_1-C_6)$-alkanoyl
$(C_6-C_{12})$-Aryloxy-$(C_1-C_6)$-alkanoyl, das im Arylteil wie oben bei Aryl beschrieben substituiert sein kann,
$(C_1-C_6)$-Acyl-$(C_1-C_8)$-alkanoyl,
Carboxy-$(C_1-C_4)$-alkanoyl,
Carbamoyl-$(C_1-C_4)$-alkanoyl,
Amino-$(C_1-C_4)$-alkyl;
$(C_1-C_4)$-Alkanoylamino-$(C_1-C_4)$-alkyl;
$(C_7-C_{13})$-Aroylamino-$(C_1-C_4)$-alkyl;
$(C_1-C_4)$-Alkoxycarbonylamino-$(C_1-C_4)$-alkyl;
$(C_6-C_{12})$-Aryl-$(C_1-C_4)$-alkoxycarbonylamino-$(C_1-C_4)$-alkyl;
$(C_6-C_{12})$-Aryl-$(C_1-C_4)$-alkylamino-$(C_1-C_4)$-alkyl;
$(C_1-C_4)$-Alkylamino-$(C_1-C_4)$-alkyl;
Di-$(C_1-C_4)$-alkylamino-$(C_1-C_4)$-alkyl;
Guanidino-$(C_1-C_4)$-alkyl;
$(C_1-C_4)$-Alkylthio-$(C_1-C_4)$-alkyl;
$(C_6-C_{12})$-Arylthio-$(C_1-C_4)$-alkyl, das im Arylteil wie oben beschrieben substituiert sein kann;
Carboxy-$(C_1-C_4)$-alkyl; Carbamoyl-$(C_1-C_4)$-alkyl;
$(C_1-C_4)$-Alkoxycarbonyl-$(C_1-C_4)$-alkyl;
$(C_6-C_{12}$-Aryloxy-$(C_1-C_4)$-alkyl, das im Arylteil wie oben bei Aryl beschrieben substituiert sein kann, bedeutet und $R^3$ und $R^4$ zusammen mit den sie tragenden Atomen ein gegebenenfalls substituiertes mono- oder bicyclisches Ringsystem von 3 - 8 C-Atomen bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, ausgenommen sind.

3. Verbindung der Formel I gemäß einem oder mehreren der Ansprüche 1 und 2 dadurch gekennzeichnet, daß
$R^1$ Wasserstoff; $(C_1-C_8)$-Alkyl; $(C_2-C_6)$-Alkenyl;
$(C_3-C_9)$-Cycloalkyl; Amino-$(C_1-C_4)$-alkyl;
$(C_2-C_5)$-Acylamino-$(C_1-C_4)$-alkyl;
$(C_7-C_{13})$-Aroylamino-$(C_1-C_4)$-alkyl;
$(C_1-C_4)$-Alkoxycarbonylamino-$(C_1-C_4)$-alkyl;
$(C_1-C_4)$-Alkoxycarbonyl-$(C_1-C_{10})$-alkyl;
Carbamoyl-$(C_1-C_{10})$-alkyl; $(C_1-C_4)$-Mono oder -Dialkylcarbamo-$(C_1-C_{10})$-alkyl, wobei Alkyl gegebenenfalls durch Phenyl substituiert ist;
$(C_6-C_{12})$-Aryl-$(C_1-C_6)$-alkyl;
$(C_6-C_{12})$-Aryl-$(C_1-C_4)$-alkoxycarbonylamino-$(C_1-C_4)$-alkyl;
$(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann,
bedeutet,
$R^2$ Wasserstoff;
$(C_1-C_6)$-Alkyl, das gegebenenfalls durch Amino, $(C_1-C_6)$-Acylamino oder Benzoylamino substituiert sein kann;
$(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl,
$(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann;

($C_6$-$C_{12}$)-Aryl-($C_1$-$C_6$)-alkyl oder ($C_7$-$C_{13}$)-Aroyl-($C_1$-$C_4$)-alkyl, die beide wie in Anspruch 2 definiert im Arylrest substituiert sein können;
($C_1$-$C_8$)-Alkanoyl;
($C_6$-$C_{12}$)-Aryl-($C_1$-$C_6$)-alkanoyl, das wie in Anspruch 2 definiert im Arylteil substituiert sein kann;
($C_1$-$C_4$)-Alkoxycarbonyl-($C_1$-$C_7$)-alkanoyl;
($C_6$-$C_{12}$)-Aryloxycarbonyl-($C_1$-$C_7$)-alkanoyl, das wie in Anspruch 2 definiert im Arylteil substituiert sein kann;
($C_1$-$C_4$)-Acyl-($C_2$-$C_6$)-alkanoyl;
($C_1$-$C_4$)-Acylamino-($C_1$-$C_6$)-alkanoyl;
($C_7$-$C_{13}$)-Aroyl-($C_1$-$C_6$)-alkanoyl, das wie in Anspruch 2 definiert im Arylteil substituiert sein kann, bedeutet;
$R^3$ und $R^4$ zusammen mit den sie tragenden Atomen, ein monocyclisches alicyclisches oder aromatisches Ringsystem, insbesondere Cyclopentan, Cyclopenten, Cyclohexan, Cycloctan und Benzol, bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, sowie Verbindungen in denen $R^2$ α-Acylamino-($C_1$-$C_6$)-alkanoyl bedeutet, ausgenommen sind.

4. Verbindung der Formel I

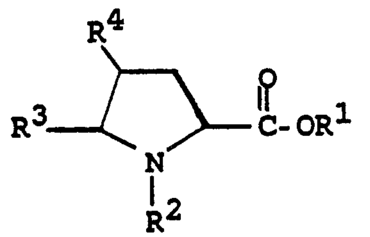

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,
einen gegebenenfalls substituierten ($C_3$-$C_8$)-Cycloalkyl-($C_1$-$C_8$)-alkanoyl-Rest,
einen gegebenenfalls substituierten ($C_7$-$C_{13}$)-Aroyl-Rest,
einen gegebenenfalls substituierten ($C_6$-$C_{12}$)-Aryl-($C_1$-$C_{18}$)-alkanoyl Rest,
einen gegebenenfalls substituierten ($C_7$-$C_{13}$)-Aroyl-($C_1$-$C_{18}$)-alkanoyl-Rest oder
einen gegebenenfalls substituierten ($C_1$-$C_{10}$)-Alkoxycarbonyl-Rest oder falls nicht von vorstehenden Definitionen schon umfaßt, die erforderlichenfalls geschützte Seitenkette einer natürlich vorkommenden α-Aminosäure bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten aromatischen Rest mit 6 - 12 C-Atomen oder
einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,

einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet, jedoch nicht Wasserstoff ist,
oder $R^4$ mit dem diesen Substituenten tragenden C-Atom ein spiroverknüpftes mono- oder bicyclisches Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze als pharmakologischer Wirkstoff.

5. Verbindung der Formel I gemäß Anspruch 4 als pharmakologischer Wirkstoff zur Behandlung und Prophylaxe cognitiver Dysfunktionen.

6. Verfahren zur Herstellung einer Verbindung der Formel I, gemäß einem oder mehreren der Ansprüche 1 bis 5, dadurch gekennzeichnet, daß man ihre Fragmente ineinem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel I mit freier(en) Carboxylgruppe(n) gegebenenfalls verestert und die erhaltenen Verbindungen gegebenenfalls in ihre physiologisch verträglichen Salze überführt.

7. Verwendung einer Verbindung der Formel I

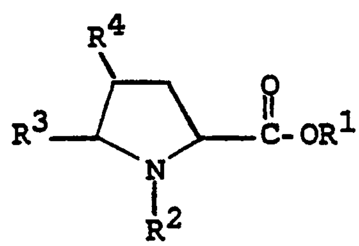

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,
einen gegebenenfalls substituierten ($C_3$-$C_8$)-Cycloalkyl-($C_1$-$C_8$)-alkanoyl-Rest,
einen gegebenenfalls substituierten ($C_7$-$C_{13}$)-Aroyl-Rest,
einen gegebenenfalls substituierten ($C_6$-$C_{12}$)-Aryl-($C_1$-$C_{18}$)-alkanoyl Rest,
einen gegebenenfalls substituierten ($C_7$-$C_{13}$)-Aroyl-($C_1$-$C_{18}$)-alkanoyl-Rest oder
einen gegebenenfalls substituierten ($C_1$-$C_{10}$)-Alkoxycarbonyl-Rest oder falls nicht von vorstehenden Definitionen schon umfaßt, die erforderlichenfalls geschützte Seitenkette einer natürlich vorkommenden α-Aminosäure bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten aromatischen Rest mit 6 - 12 C-Atomen oder

einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet, jedoch nicht Wasserstoff ist,
oder $R^4$ mit dem diesen Substituenten tragenden C-Atom ein spiroverknüpftes mono- oder bicyclisches Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze zur Behandlung und Prophylaxe cognitiver Dysfunktionen.

8. Pharmazeutisches Mittel enthaltend eine wirksame Menge einer Verbindung der Formel I gemäß einem oder mehreren der Ansprüche 1 bis 5 und einen physiologisch unbedenklichen Träger.

9. Verfahren zur Herstellung eines pharmazeutischen Mittels gemäß Anspruch 8, dadurch gekennzeichnet, daß man den Wirkstoff zusammen mit einem Träger und gegebenenfalls weiteren Zusatz- udn/oder Hilfsstoffen in eine geeignete Darreichungsform bringt.

Patentanspruche für folgende Vertragsstaaten: ES: GR:

1. Verfahren zur Herstellung einer Verbindung der Formel I

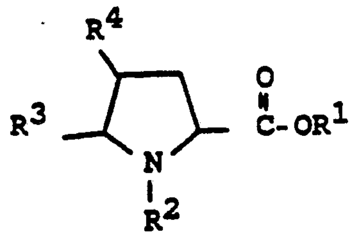

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,
einen gegebenenfalls substituierten ($C_3$-$C_8$)-Cycloalkyl-($C_1$-$C_8$)-alkanoyl-Rest,
einen gegebenenfalls substituierten ($C_7$-$C_{13}$)-Aroyl-Rest,
einen gegebenenfalls substituierten ($C_6$-$C_{12}$)-Aryl-($C_1$-$C_{18}$)-alkanoyl Rest,
einen gegebenenfalls substituierten ($C_7$-$C_{13}$)-Aroyl-($C_1$-$C_{18}$)-alkanoyl-Rest oder
einen gegebenenfalls substituierten ($C_1$-$C_{10}$)-Alkoxycarbonyl-Rest
bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,

einen gegebenenfalls substituierten aromatischen Rest mit 6 - 12 C-Atomen oder
einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
jedoch nicht Wasserstoff ist,
oder $R^4$ mit dem diesen Substituenten tragenden C-Atom ein spiroverknüpftes mono- oder bicyclisches Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, sowie Verbindungen, in denen $R^2$ gegebenenfalls substituiertes $\alpha$-Amino-($C_1$-$C_6$)-alkanoyl bedeutet, ausgenommen sind, dadurch gekennzeichnet, daß man ihr Fragmente in einem geeigneten Lösungsmittel gegebenefalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel I mit freier(en) Carboxylgruppe(n) gegebenenfalls verestert und die erhaltenen Verbindungen gegebenenfalls in ihre physiologisch verträglichen Salze überführt.

2. Verfahren gemäß Anspruch 1, dadurch gekennzeichnet, daß
$R^1$ Wasserstoff;
($C_1$-$C_{18}$)-Alkyl;
einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;
($C_6$-$C_{12}$)-Aryl, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann;
Amino-($C_1$-$C_8$)-alkyl;
($C_1$-$C_4$)-Alkanoylamino-($C_1$-$C_8$)-alkyl;
($C_7$-$C_{13}$)-Aroylamino-($C_1$-$C_8$)-alkyl;
$C_1$-$C_4$)-Alkoxycarbonylamino-($C_1$-$C_8$)-alkyl;
($C_6$-$C_{12}$)-Aryl-($C_1$-$C_4$)-alkoxycarbonylamino-($C_1$-$C_8$)-alkyl;
($C_6$-$C_{12}$)-Aryl-($C_1$-$C_4$)-alkylamino-($C_1$-$C_8$)-alkyl;
($C_1$-$C_4$)-Alkylamino-($C_1$-$C_8$)-alkyl;
Di-($C_1$-$C_4$)-alkylamino-($C_1$-$C_8$)-alkyl;
Guanidino-($C_1$-$C_8$)-alkyl;
($C_1$-$C_4$)-Alkylthio-($C_1$-$C_8$)-alkyl;
($C_6$-$C_{12}$)-Arylthio-($C_1$-$C_8$)-alkyl oder ($C_6$-$C_{12}$)-Aryloxy-($C_1$-$C_8$)-alkyl, die beide im Arylteil wie oben beschrieben substituiert sein können;
Carboxy-($C_1$-$C_{17}$)-alkyl, ($C_1$-$C_4$)-Alkoxycarbonyl-($C_1$-$C_{17}$)-alkyl, Carbamoyl-($C_1$-$C_{17}$)-alkyl, ($C_1$-$C_4$)-Mono- oder di-alkylcarbamoyl-($C_1$-$C_{17}$)-alkyl, wobei Alkyl gegebenenfalls durch ($C_6$-$C_{12}$)-Aryl substituiert ist, bedeutet;
$R^2$ Wasserstoff;
($C_1$-$C_{18}$)-Alkyl;
einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenwasserstoffrest der Formel $C_cH_{2c-d-1}$, worin c für eine ganze Zahl 3 bis 20 und d für eine gerade Zahl 0 bis (c-2) stehen;
($C_6$-$C_{12}$)-Aryl;
($C_6$-$C_{12}$)-Aryl-($C_1$-$C_8$)-alkyl oder ($C_7$-$C_{13}$)-Aroyl-($C_1$-$C_8$)-alkyl, die im Arylteil wie oben beschrieben substituiert sein können;

mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroarylteil wie oben bei Aryl beschrieben substituiert sein können;
($C_1$-$C_{18}$)-Alkanoyl;
($C_6$-$C_{12}$)-Aryl-($C_1$-$C_8$)-alkanoyl, das im Arylteil wie oben beschrieben substituiert sein kann,
($C_7$-$C_{13}$)-Aroyl-($C_1$-$C_8$)-alkanoyl, das im Arylteil wie oben beschrieben substituiert sein kann,
($C_1$-$C_6$)-Alkoxycarbonyl-($C_1$-$C_8$)alkanoyl,
($C_6$-$C_{12}$)-Aryloxycarbonyl-($C_1$-$C_8$)alkanoyl, das im Arylteil wie oben beschrieben substituiert sein kann;
($C_6$-$C_{12}$)-Aryl-($C_1$-$C_8$)-alkoxycarbonyl;
($C_1$-$C_6$)-Alkoxy-($C_1$-$C_6$)-alkanoyl
($C_6$-$C_{12}$)-Aryloxy-($C_1$-$C_6$)-alkanoyl, das im Arylteil wie oben bei Aryl beschrieben substituiert sein kann,
($C_1$-$C_6$)-Acyl-($C_1$-$C_8$)-alkanoyl,
Carboxy-($C_1$-$C_4$)-alkanoyl,
Carbamoyl-($C_1$-$C_4$)-alkanoyl,
Amino-($C_1$-$C_4$)-alkyl;
($C_1$-$C_4$)-Alkanoylamino-($C_1$-$C_4$)-alkyl;
($C_7$-$C_{13}$)-Aroylamino-($C_1$-$C_4$)-alkyl;
($C_1C_4$)-Alkoxycarbonylamino-($C_1$-$C_4$)-alkyl;
$C_6$-$C_{12}$)-Aryl-($C_1$-$C_4$)-alkoxycarbonylamino-($C_1$-$C_4$)-alkyl;
($C_6$-$C_{12}$)-Aryl-($C_1$-$C_4$)-alkylamino-($C_1$-$C_4$)-alkyl;
($C_1$-$C_4$)-Alkylamino-($C_1$-$C_4$)-alkyl;
Di-($C_1$-$C_4$)-alkylamino-($C_1$-$C_4$)-alkyl;
Guanidino-($C_1$-$C_4$)-alkyl;
($C_1$-$C_4$)-Alkylthio-($C_1$-$C_4$)-alkyl;
($C_6$-$C_{12}$)-Arylthio-($C_1$-$C_4$)-alkyl, das im Arylteil wie oben beschrieben substituiert sein kann;
Carboxy-($C_1$-$C_4$)-alkyl; Carbamoyl-($C_1$-$C_4$)-alkyl;
($C_1$-$C_4$)-Alkoxycarbonyl-($C_1$-$C_4$)-alkyl;
($C_6$-$C_{12}$-Aryloxy-($C_1$-$C_4$)-alkyl, das im Arylteil wie oben bei Aryl beschrieben substituiert sein kann, bedeutet und $R^3$ und $R^4$ zusammen mit den sie tragenden Atomen ein gegebenenfalls substituiertes mono- oder bicyclisches Ringsystem von 3 - 8 C-Atomen bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, ausgenommen sind.

3. Verfahren gemäß Anspruch 1 und/oder 2, dadurch gekennzeichnet, daß
$R^1$ Wasserstoff; ($C_1$-$C_8$)-Alkyl; ($C_2$-$C_6$)-Alkenyl;
($C_3$-$C_9$)-Cycloalkyl; Amino-($C_1$-$C_4$)-alkyl;
($C_2$-$C_5$)-Acylamino-($C_1$-$C_4$)-alkyl;
($C_7$-$C_{13}$)-Aroylamino-($C_1$-$C_4$)-alkyl;
($C_1$-$C_4$)-Alkoxycarbonylamino-($C_1$-$C_4$)-alkyl;
($C_1$-$C_4$)-Alkoxycarbonyl-($C_1$-$C_{10}$)-alkyl;
Carbamoyl-($C_1$-$C_{10}$)-alkyl; ($C_1$-$C_4$)-Mono oder -Dialkylcarbamo-($C_1$-$C_{10}$)-alkyl, wobei Alkyl gegebenenfalls durch Phenyl substituiert ist;
($C_6$-$C_{12}$)-Aryl-($C_1$-$C_6$)-alkyl;
($C_6$-$C_{12}$)-Aryl-($C_1$-$C_4$)-alkoxycarbonylamino-($C_1$-$C_4$)-alkyl;
($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann,
bedeutet,
$R^2$ Wasserstoff;
($C_1$-$C_6$)-Alkyl, das gegebenenfalls durch Amino, ($C_1$-$C_6$)-Acylamino oder Benzoylamino substituiert sein kann; ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl,
($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann;
($C_6$-$C_{12}$)-Aryl-($C_1$-$C_6$)-alkyl oder ($C_7$-$C_{13}$)-Aroyl-($C_1$-$C_4$)-alkyl, die beide wie in Anspruch 2 definiert im Arylrest substituiert sein können;
($C_1$-$C_8$)-Alkanoyl;
($C_6$-$C_{12}$)-Aryl-($C_1$-$C_6$)-alkanoyl, das wie in Anspruch 2 definiert im Arylteil substituiert sein kann;
($C_1$-$C_4$)-Alkoxycarbonyl-($C_1$-$C_7$)-alkanoyl;

$(C_6-C_{12})$-Aryloxycarbonyl-$(C_1-C_7)$-alkanoyl, das wie in Anspruch 2 definiert im Arylteil substituiert sein kann;
$(C_1-C_4)$-Acyl-$(C_2-C_6)$-alkanoyl;
$(C_1-C_4)$-Acylamino-$(C_1-C_6)$-alkanoyl;
$(C_7-C_{13})$-Aroyl-$(C_1-C_6)$-alkanoyl, das wie in Anspruch 2 definiert im Arylteil substituiert sein kann, bedeutet;
$R^3$ und $R^4$ zusammen mit den sie tragenden Atomen, ein monocyclisches alicyclisches oder aromatisches Ringsystem, insbesondere Cyclopentan, Cyclopenten, Cyclohexan, Cycloctan und Benzol, bilden, sowie deren physiologisch verträgliche Salze, wobei Verbindungen, in denen gleichzeitig $R^1$ Wasserstoff, Benzyl oder n-Octyl, $R^2$ tert.-Butoxycarbonyl und $R^3$ und $R^4$ zusammen mit den sie tragenden Atomen den Cyclopentan- oder Cyclopentenring bedeuten, sowie Verbindungen in denen $R^2$ α-Acylamino-$(C_1-C_6)$-alkanoyl bedeutet, ausgenommen sind.

4. Verwendung einer Verbindung der Formel I

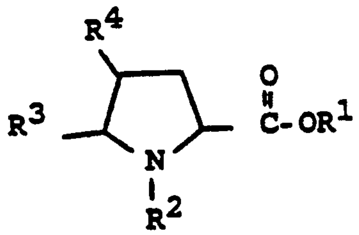

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen,
einen gegebenenfalls substituierten Alkanoyl-Rest mit 1 - 18 C-Atomen,
einen gegebenenfalls substituierten $(C_3-C_8)$-Cycloalkyl-$(C_1-C_8)$-alkanoyl-Rest,
einen gegebenenfalls substituierten $(C_7-C_{13})$-Aroyl-Rest,
einen gegebenenfalls substituierten $(C_6-C_{12})$-Aryl-$(C_1-C_{18})$-alkanoyl Rest,
einen gegebenenfalls substituierten $(C_7-C_{13})$-Aroyl-$(C_1-C_{18})$-alkanoyl-Rest oder
einen gegebenenfalls substituierten $(C_1-C_{10})$-Alkoxycarbonyl-Rest oder falls nicht von vorstehenden Definitionen schon umfaßt, die erforderlichenfalls geschützte Seitenkette einer natürlich vorkommenden α-Aminosäure bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten aromatischen Rest mit 6 - 12 C-Atomen oder
einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen bedeutet, jedoch nicht Wasserstoff ist,
oder $R^4$ mit dem diesen Substituenten tragenden C-Atom ein spiroverknüpftes mono- oder bicyclisches

Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze zur Behandlung und Prophylaxe cognitiver Dysfunktion.

5. Verfahren zur Herstellung einer pharmazeutischen Zubereitung enthaltend eine Verbindung der Formel I

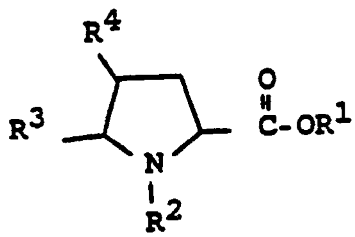

(I)

in welcher
$R^1$ Wasserstoff,
einen gegebenenfalls, substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen,
einen gegebenenfalls substituierten Alkanoyl-Rest mit 1 - 18 C-Atomen,
einen gegebenenfalls substituierten ($C_3$-$C_8$)-Cycloalkyl-($C_1$-$C_8$)-alkanoyl-Rest,
einen gegebenenfalls substituierten ($C_7$-$C_{13}$)-Aroyl-Rest,
einen gegebenenfalls substituierten ($C_6$-$C_{12}$)-Aryl-($C_1$-$C_{18}$)-alkanoyl Rest,
einen gegebenenfalls substituierten ($C_7$-$C_{13}$)-Aroyl-($C_1$-$C_{18}$)-alkanoyl-Rest oder
einen gegebenenfalls substituierten ($C_1$-$C_{10}$)-Alkoxycarbonyl-Rest oder falls nicht von vorstehenden Definitionen schon umfaßt, die erforderlichenfalls geschützte Seitenkette einer natürlich vorkommenden $\alpha$-Aminosäure bedeutet,
$R^3$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten aromatischen Rest mit 6 - 12 C-Atomen oder
einen gegebenenfalls substituierten araliphatischen Rest mit 7 - 32 C-Atomen bedeutet,
$R^4$ einen gegebenenfalls substituierten aliphatischen Rest mit 1 - 18 C-Atomen,
einen gegebenenfalls substituierten alicyclischen Rest mit 3 - 20 C-Atomen,
einen gegebenenfalls substituierten alicyclisch-aliphatischen Rest mit 4 - 20 Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet, jedoch nicht Wasserstoff ist,
oder $R^4$ mit dem diesen Substituenten tragenden C-Atom ein spiroverknüpftes mono- oder bicyclisches Ringsystem von 3 - 8 C-Atomen bildet,
oder $R^3$ und $R^4$ zusammen mit den sie tragenden Atomen ein gegebenenfalls substituieres mono- oder bicyclisches Ringsystem von 3 - 8 C-Atomen bilden, sowie deren physiologisch verträgliche Salze dadurch gekennzeichnet, daß man diese zusammen mit einem physiologisch unbedenklichen Träger und gegebenefalls weiteren Hilfs-und Zusatzstoffen in eine geeignete Darreichungsform bringt.